# EUROPEAN PATENT SPECIFICATION

(11) **EP 2 584 039 B1**
(45) Date of publication and mention of the grant of the patent: **10.02.2016**
(21) Application number: 10853305.0
(22) Date of filing: 08.12.2010
(51) Int. Cl.: C12Q 1/68

(54) **SNP FOR PREDICTING THE SENSITIVITY TO ANTICANCER TARGETED THERAPEUTIC FORMULATION**
SNP ZUR VORHERSAGE DER SENSITIVITÄT GEGENÜBER EINER THERAPEUTISCHEN ANTIKREBSFORMULIERUNG
SNP DESTINÉ À PRÉDIRE LA SENSIBILITÉ À UNE FORMULATION THÉRAPEUTIQUE ANTICANCÉREUSE CIBLÉE

(30) Priority: 15.06.2010 KR 20100056279
(43) Date of publication of application: 24.04.2013
(73) Proprietor: The Asan Foundation, Songpa-gu, Seoul 138-736 (KR); Korea Research Institute of Bioscience and Biotechnology, Daejeon 305-806 (KR)
(72) Inventor: KIM, Jin Cheon, Seoul 135-837 (KR); KIM, Yong Sung, Daejeon 305-806 (KR); KIM, Seon Young, Daejeon 305-806 (KR); CHO, Dong Hyung, Seoul 138-783 (KR)
(74) Representative: Wells, Andrew
(86) International application number: PCT/KR2010/008734
(87) International publication number: WO 2011/159004

(56) References cited:
- "Reference SNP(refSNP) Cluster Report: rs2230392", , 29 August 2001 (2001-08-29), XP055086498, Retrieved from the Internet: URL:http://www.ncbi.nlm.nih.gov/projects/S NP/snp_ref.cgi?searchType=adhoc_search&typ e=rs&rs=rs2230392 [retrieved on 2013-11-04]
- Snpdev: "Reference SNP(refSNP) Cluster Report: rs1534443", , 20 October 2000 (2000-10-20), XP055086504, Retrieved from the Internet: URL:http://www.ncbi.nlm.nih.gov/projects/S NP/snp_ref.cgi?rs=1534443 [retrieved on 2013-11-04]
- Snpdev: "Reference SNP(refSNP) Cluster Report: rs13321", , 23 August 1999 (1999-08-23), XP055086507, Retrieved from the Internet: URL:http://www.ncbi.nlm.nih.gov/projects/S NP/snp_ref.cgi?rs=13321 [retrieved on 2013-11-04]
- Snpdev: "Reference SNP(refSNP) Cluster Report: rs1049550 **Clinical Channel**", , 13 September 2000 (2000-09-13), XP055086508, Retrieved from the Internet: URL:http://www.ncbi.nlm.nih.gov/projects/S NP/snp_ref.cgi?rs=1049550 [retrieved on 2013-11-04]
- Snpdev: "Reference SNP(refSNP) Cluster Report: rs11247226", , 18 November 2003 (2003-11-18), XP055086510, Retrieved from the Internet: URL:http://www.ncbi.nlm.nih.gov/SNP/snp_re f.cgi?rs=rs11247226 [retrieved on 2013-11-04]
- Snpdev: "Reference SNP(refSNP) Cluster Report: rs733743", , 6 September 2000 (2000-09-06), XP055086513, Retrieved from the Internet: URL:http://www.ncbi.nlm.nih.gov/SNP/snp_re f.cgi?searchType=adhoc_search&type=rs&rs=r s733743 [retrieved on 2013-11-04]
- Snpdev: "Reference SNP(refSNP) Cluster Report: rs4932305", , 12 February 2003 (2003-02-12), XP055086514, Retrieved from the Internet: URL:http://www.ncbi.nlm.nih.gov/projects/S NP/snp_ref.cgi?rs=rs4932305 [retrieved on 2013-11-04]
- Snpdev: "Reference SNP(refSNP) Cluster Report: rs2274159 ** With non-pathogenic allele **", , 5 September 2001 (2001-09-05), XP055086515, Retrieved from the Internet: URL:http://www.ncbi.nlm.nih.gov/projects/S NP/snp_ref.cgi?searchType=adhoc_search&typ e=rs&rs=rs2274159 [retrieved on 2013-11-04]
- Snpdev: "Reference SNP(refSNP) Cluster Report: rs361863", , 5 July 2000 (2000-07-05), XP055086516, Retrieved from the Internet: URL:http://www.ncbi.nlm.nih.gov/SNP/snp_re f.cgi?type=rs&rs=361863 [retrieved on 2013-11-04]
- Snpdev: "Reference SNP(refSNP) Cluster Report: rs3729740", , 12 July 2002 (2002-07-12), XP055086542, Retrieved from the Internet: URL:http://www.ncbi.nlm.nih.gov/projects/S NP/snp_ref.cgi?rs=rs3729740 [retrieved on 2013-11-04]
- Snpdev: "Reference SNP(refSNP) Cluster Report: rs2306393", , 5 September 2001 (2001-09-05), XP055086544, Retrieved from the Internet: URL:http://www.ncbi.nlm.nih.gov/SNP/snp_re f.cgi?rs=rs2306393 [retrieved on 2013-11-04]
- "Genome-Wide Human SNP Array 6.0", INTERNET CITATION, 26 March 2009 (2009-03-26), pages 1-4, XP002698985, Retrieved from the Internet: URL:http://revistas.usc.es/export/sites/de fault/cegen/descargas/Affymetrix/GeneChip_ datasheet/genomewide_human_SNP_6.0.pdf [retrieved on 2013-06-18]
- A. M. SCHULTHEIS ET AL: "Polymorphisms and Clinical Outcome in Recurrent Ovarian Cancer Treated with Cyclophosphamide and Bevacizumab", CLINICAL CANCER RESEARCH, vol. 14, no. 22, 15 November 2008 (2008-11-15), pages 7554-7563, XP055086458, ISSN: 1078-0432, DOI: 10.1158/1078-0432.CCR-08-0351
- TORBEN FRÃ Â STRUP HANSEN ET AL: "Microvessel density and the association with single nucleotide polymorphisms of the vascular endothelial growth factor receptor 2 in patients with colorectal cancer", VIRCHOWS ARCHIV, SPRINGER, BERLIN, DE, vol. 456, no. 3, 9 February 2010 (2010-02-09), pages 251-260, XP019803138, ISSN: 1432-2307
- HSU JERRY Y ET AL: "Monoclonal antibodies targeting vascular endothelial growth factor: current status and future challenges in cancer therapy", BIODRUGS: CLINICAL IMMUNOTHERAPEUTICS, BIOPHARMACEUTICALS AND GENE THERAPY, ADIS INTERNATIONAL, FR, vol. 23, no. 5, 1 January 2009 (2009-01-01), pages 289-304, XP009129983, ISSN: 1173-8804
- GAMAZON, E.R. ET AL.: 'Chemotherapeutic drug susceptibility associated SNPs are enriched in expression quantitative trait loci' PROC. NATL. ACAD. SCI. USA vol. 107, no. 20, 18 May 2010, pages 9287 - 9292
- STOCCO, G. ET AL.: 'Genetic polymorphism of inosine-triphosphate-pyrophosphatase influences mercaptopurine metabolism and toxicity during treatment of acute lymphoblastic leukemia individualized for thiopurine-S-methyl-transferase status' EXPERT OPIN. DRUG SAF. vol. 9, no. 1, January 2010, pages 23 - 37
- DAVIDSEN, M.L. ET AL.: 'Pharmacogenetics influence treatment efficacy in childhood acute lymphoblastic leukemia' J. PEDIATR. HEMATOL. ONCOL. vol. 30, no. 11, November 2008, pages 831 - 849
- HASEGAWA, T. ET AL.: 'Analysis of single nucleotide polymorphisms in uridine/cytidine kinase gene encoding metabolic enzyme of 3'-ethynylcytidine' NUCLEIC ACIDS RES. vol. 2, 2002, pages 237 - 238
- HENRIETTE TANJA L ET AL: "Pharmacogenetics in chemotherapy of colorectal cancer", BAILLIERE'S BEST PRACTICE AND RESEARCH. CLINICAL GASTROENTEROLOGY, BAILLIERE TINDALL, LONDON, US, vol. 23, no. 2, 1 April 2009 (2009-04-01), pages 257-273, XP026105371, ISSN: 1521-6918, DOI: 10.1016/J.BPG.2009.02.011 [retrieved on 2009-05-04]

## Description

### [Technical Field]

The present disclosure relates to a single nucleotide polymorphism (SNP) marker for predicting the sensitivity to an anticancer targeted drug, a polynucleotide containing the same, and a method for predicting the sensitivity to a specific anticancer targeted drug.

### [Background Art]

Various genetic differences between individuals are most attributable to single nucleotide polymorphisms, and some genotypes exhibit functional differences in corresponding proteins and their metabolism, which is known to be susceptible to pharmacogenetic individual sensitivities (Huang and Ratain, CA Cancer J Clin 2009;59:42-55).
Meanwhile, many anticancer drugs are used as effective therapeutic agents, but a new problem is the resistance of cancer cells (Tsuruo et al., 1985). The resistance to anticancer drugs is caused by various mechanisms in which cells exposed to drugs due to long-term use of anticancer drugs reduce the accumulation of drugs in cells (Shen et al., 1986; Shen et al., 2000; Gottesman et al., 2002), activate detoxification or degradation (Schuetz et al., 1999; Goto et al., 2000), or modify targeted proteins (Urasaki et al., 2001). These processes are the most significant obstacle in the treatment of cancers and closely related to treatment failures (Kang, 1996). Among various mechanisms involved in the resistance to drugs, multidrug resistance (MDR) plays an important role (Brooks et al., 2003). Multidrug resistance refers to a phenomenon that causes the resistance to various anticancer drugs with entirely different structures or functions as well as to anticancer drugs being used (Mehta et al., 1992; Gottesman, 2000). If a specific anticancer drug does not take effect when chemotherapy is given to a cancer patient, the resistance is often developing to other anticancer drugs later, and it can be often observed that there is no therapeutic effect even though complex chemotherapy, in which various types of anticancer drugs with different mechanisms of action are administered at the same time during initial treatment, is given. As a result, the limited range of available anticancer drugs is recognized as an important problem in the chemotherapy of cancers. Accordingly, the selection using suitable therapeutic response markers can lead to a significant progress in the treatment with anticancer drugs. Thus, extensive research on the therapeutic response to individual anticancer drugs according to SNP genotype has recently been continuously carried out.

Schultheis A.M. et al, Clinical Cancer Research (2008), vol. 14, no. 22, pp7554-7563, is concerned with polymorphisms and clinical outcome in recurrent ovarian cancer treated with cyclophosphamide and bevacizumab.

Hansen T.F. et al, Virchows Archiv, Springer, Berlin, DE (2010), vol. 456, no. 3, pp251-260, is concerned with microvessel density and the association with single nucleotide polymorphisms of the Vascular Endothelial Growth Factor Receptor 2 in patients with colorectal cancer.

Hsu, J.Y., Biodrugs: Clinical Immunotherapeutics, Biopharmaceuticals and Gene Therapy, Adis International, FR, (2009), vol. 23, no. 5, pp289-304, provides a review of monoclonal antibodies targeting Vascular Endothelial Growth Factor.

Henriette T. L. et al, Best practice & research. Clinical gastroenterology (2009), vol. 23, no. 2, pp 257-273, is concerned with polymorphisms in VEGF and clinical outcome in colorectal cancer.

However, due to the complex interaction of biological response-related factors to a specific drug, the diversity of therapeutic agents and administration routes, and the difficulty in securing massive amounts of samples, there is no remarkable achievement yet.

### [Disclosure]

### [Technical Problem]

To make up for these weaknesses, the present inventors have determined the drug response of tumors using an *in vitro* tumor response assay, selected a result from lymphocyte DNA of the same patient by association analysis, and discovered SNP markers for predicting the sensitivity to specific anticancer targeted drugs based on human genetic information and population genetic analysis (Korean Patent Application No. 10-2009-0130540.

Accordingly, an object of the present invention is to provide a single nucleotide polymorphism (SNP) marker for predicting the sensitivity to an anticancer targeted drug.

Another object of the present invention is to provide a method for predicting the sensitivity to a specific anticancer targeted drug.

### [Technical Solution]

To achieve the above objects, the present invention provides a method for predicting sensitivity of a subject having colorectal cancer to anticancer targeted drugs selected from at least one of bevacizumab and FOLFIRI (5-FU + leucovorin + irinotecan), the method comprising:
determining the type of a nucleotide at the position of the SNP corresponding to the nucleotide at position 401 of SEQ ID NO: 4 from nucleic acid molecules isolated from the subject.

In one aspect, when the nucleotide at the position of the SNP, corresponding to the nucleotide at position 401 of SEQ ID NO: 4, is determined as T, it is predicted that the sensitivity to at least one of bevacizumab and FOLFIRI (5-FU + leucovorin + irinotecan), anticancer targeted drugs, is high.

The invention further provides use of a polynucleotide or a complementary polynucleotide thereof, selected from polynucleotides comprising at least 8 contiguous nucleotides including a nucleotide at position 401 of SEQ ID NO: 4, in a method of the invention. The at least 8 contiguous nucleotides may be 8 to 100 contiguous nucleotides.

The invention also provides use of a primer or probe in a method of the invention wherein the primer or probe comprises the polynucleotide or complementary polynucleotide whose use is described above.

The invention also provides use of a microarray, or a kit comprising said microarray, in a method of the invention, wherein the microarray comprises the polynucleotide whose use is described above, a polypeptide encoded by the same, or a cDNA thereof.
Described herein is a polynucleotide, or a complementary polynucleotide thereof, for predicting sensitivity to one or more anticancer targeted drugs, selected from polynucleotides comprising at least 8 contiguous nucleotides including each nucleotide at a specific position of the following SEQ ID NOS: 1 to 11.

The nucleotide at a specific position may be a nucleotide at position 201 of one of SEQ ID NOS: 1, 7, and 10, a nucleotide at position 251 of SEQ ID NO: 3, a nucleotide at position 301 of one of SEQ ID NOS: 5 and 11, a nucleotide at position 401 of one of SEQ ID NOS: 4, 6, 8, 9, and a nucleotide at position 406 of SEQ ID NO: 2.

The at least 8 contiguous nucleotides may be 8 to 100 contiguous nucleotides.

Moreover, also described is a primer or probe for predicting sensitivity to anticancer targeted drugs, the primer comprising the polynucleotide or complementary polynucleotide thereof.

Further described is a microarray for predicting sensitivity to anticancer targeted drugs, the microarray comprising the polynucleotide, a polypeptide encoded by the same, or a cDNA thereof.

In addition, also described is a kit for predicting sensitivity to anticancer targeted drugs, the kit comprising the microarray.

Additionally, the present disclosure relates to a method for predicting sensitivity to anticancer targeted drugs, the method comprising the steps of: (1) isolating nucleic acid molecules from a subject; and (2) determining the type of a nucleotide at the position of SNP of a polynucleotide comprising SEQ ID NOS: 1 to 11 from the isolated nucleic acid molecules.

In step (2) of the prediction method, when the nucleotide at the position of the SNP, corresponding to the nucleotide at position 201 of SEQ ID NO: 1, is determined as A, it may be predicted that the sensitivity to bevacizumab, an anticancer targeted drug, is high.

In step (2) of the prediction method, when the nucleotide at the position of the SNP, corresponding to the nucleotide at position 406 of SEQ ID NO: 2, is determined as T, when the nucleotide at the position of the SNP, corresponding to the nucleotide at position 251 of SEQ ID NO: 3, is determined as C, or when the nucleotide at the position of the SNP, corresponding to the nucleotide at position 401 of SEQ ID NO: 4, is determined as T, it may be predicted that the sensitivity to at least one of bevacizumab and FOLFIRI (5-FU + leucovorin + irinotecan), anticancer targeted drugs, is high.

In step (2) of the prediction method, when the nucleotide at the position of the SNP, corresponding to the nucleotide at position 301 of SEQ ID NO: 5, is determined as G, it may be predicted that the sensitivity to at least one of bevacizumab and FOLFOX (5-FU + leucovorin + oxaliplatin), anticancer targeted drugs, is high.

In step (2) of the prediction method, when the nucleotide at the position of the SNP, corresponding to the nucleotide at position 401 of SEQ ID NO: 6, is determined as G, it may be predicted that the sensitivity to cetuximab, an anticancer targeted drug, is high.

In step (2) of the prediction method, when the nucleotide at the position of the SNP, corresponding to the nucleotide at position 201 of SEQ ID NO: 7, is determined as A, when the nucleotide at the position of the SNP, corresponding to the nucleotide at position 401 of SEQ ID NO: 8, is determined as G, or when the nucleotide at the position of the SNP, corresponding to the nucleotide at position 401 of SEQ ID NO: 9, is determined as C, it may be predicted that the sensitivity to at least one of cetuximab and FOLFIRI (5-FU + leucovorin + irinotecan), anticancer targeted drugs, is high.

In step (2) of the prediction method, when the nucleotide at the position of the SNP, corresponding to the nucleotide at position 201 of SEQ ID NO: 10, is determined as G, or when the nucleotide at the position of the SNP, corresponding to the nucleotide at position 301 of SEQ ID NO: 11, is determined as T, it may be predicted that the sensitivity to at least one of cetuximab and FOLFOX (5-FU + leucovorin + oxaliplatin), anticancer targeted drugs, is high.

### [Advantageous Effects]

The present disclosure enables to predict useful drugs against tumors that are resistant to drugs using a small amount of blood sample from a patient, enables to select the most suitable anticancer targeted drug over the entire therapeutic regimen of a metastatic or recurrent cancer patient, and has developed an appropriate diagnosis tool.

Moreover, the present disclosure enables to newly discover SNP markers that can predict the response to anticancer drugs and common therapeutic agents using drugs including new drugs continuously in the future and can extend the application range.

### [Description of Drawings]

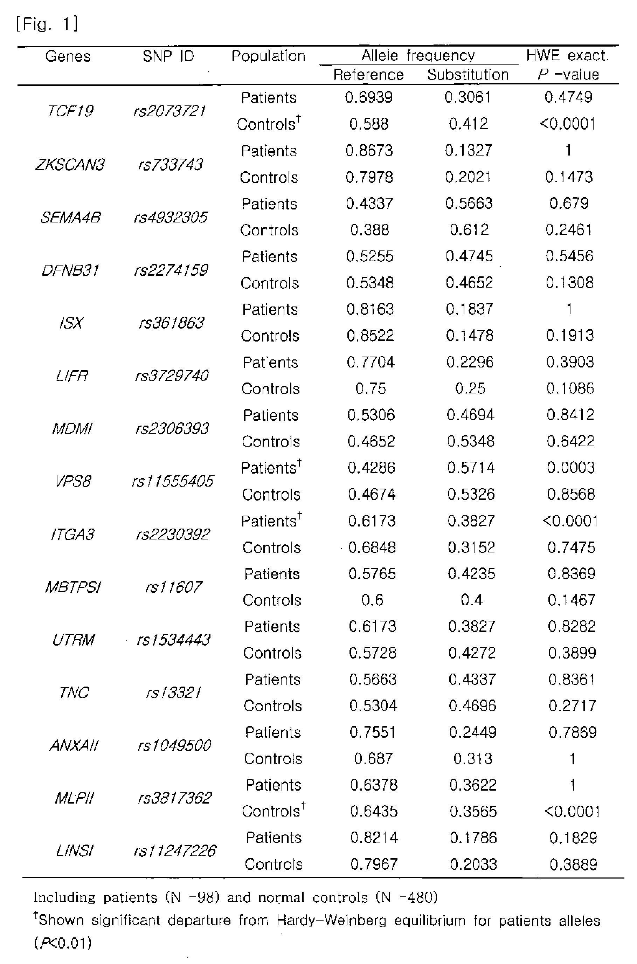

FIG. 1 shows 15 candidate SNPs of selected 15 types of genes sensitive to 6 types of anticancer targeted agents, in which TCF19 rs2073721 and MLPH rs3817362 deviate from Hardy-Weinberg equilibrium in a normal population and thus are excluded from the candidate SNPs.

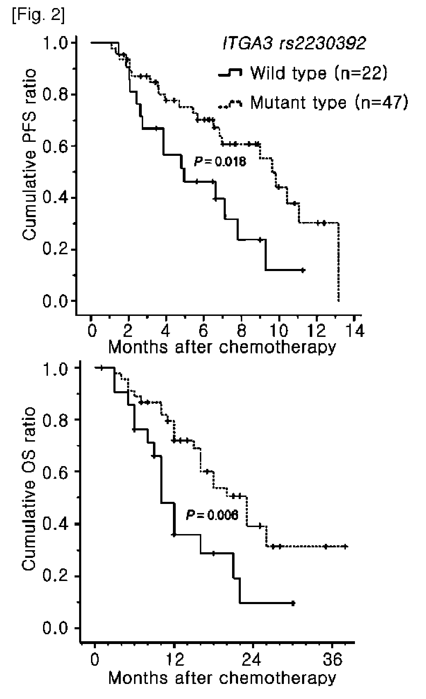

FIG. 2 shows the results of association analysis on bevacizumab-sensitive SNP genotypes in colorectal cancer patients whose treatment with the corresponding drugs has been terminated in clinics. Metastatic and recurrent cancer patients treated with bevacizumab, an anticancer targeted drug, and carrying homozygous or heterozygous substitution alleles of ITGA3 rs2230392 SNP exhibit greater sensitivity and longer progression-free survival and overall survival than those with homozygous reference alleles.

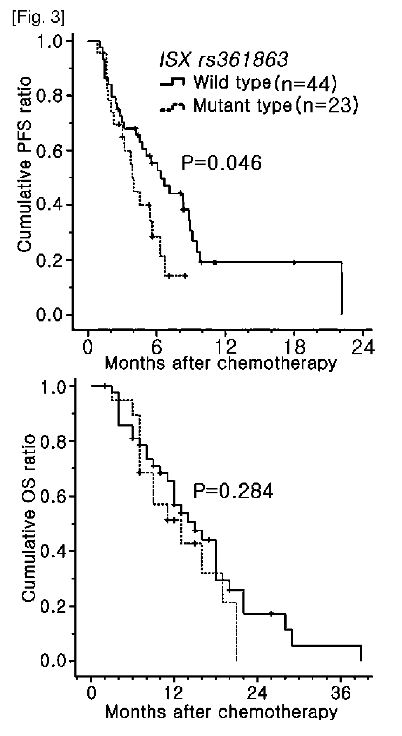

FIG. 3 shows the results of association analysis on cetuximab-sensitive SNP genotypes on colorectal cancer patients whose treatment with the corresponding drugs has been terminated in clinics. Metastatic and recurrent cancer patients treated with cetuximab, an anticancer targeted drug, and carrying homozygous reference alleles of ISX rs361863 SNP exhibit greater sensitivity and longer progression-free survival than those with homozygous or heterozygous substitution alleles.

### [Mode for Disclosure]

The present disclosure relates to single nucleotide polymorphism (SNP) markers for predicting the sensitivity to anticancer targeted drugs.

The SNPs may be screened by the method disclosed in Korean Patent Application No. 10-2009-0130540. More specifically, the SNPs for predicting the sensitivity to anticancer targeted drugs may be screened by a method comprising the steps of:
(1) selecting candidate SNP genotypes for predicting the sensitivity to anticancer targeted drugs by performing association analysis on the result of an *in vitro* chemosensitivity assay using patients tumor tissues and the result of a SNP analysis on the same patients; and
(2) selecting SNP genotypes from the selected genotypes having a nominal P value is 1% or less based on the frequency of alleles in Asian genotypes, genotypes located in the linkage disequilibrium block, haplotype tagging SNPs (htSNPs), functional SNPs, and Hardy-Weinberg equilibrium P values in a normal population.

In the above method, the *in vitro* chemosensitivity assay of step (1) may be performed by the method disclosed in Korean Patent Application No. 10-2008-0115296. The *in vitro* chemosensitivity assay may comprise the steps of:
(i) preparing a primary anti-cancer drug reaction plate and a secondary anti-cancer drug reaction plate;
(ii) injecting a tumor tissue sample prepared from a subject into reaction wells of the primary anti-cancer drug reaction plate and reaction wells of the secondary anti-cancer drug reaction plate and culturing;
(iii) treating and reacting primary anti-cancer drug candidates with the reaction wells of the primary reaction plate and the reaction wells of the secondary reaction plate;
(iv) measuring and analyzing the sensitivity of the primary anti-cancer drug candidates treated with the reaction wells of the primary anti-cancer drug reaction plate and treating and reacting secondary anti-cancer drug candidates, which have a combination different from that of the primary anti-cancer drug, with the reaction wells of the secondary anti-cancer drug reaction plate; and
(v) measuring and analyzing the sensitivity of the secondary anti-cancer drug candidates applied to the reaction wells of the secondary anti-cancer drug reaction plate, and the method can screen even the sensitivity of new drugs without analysis on clinical outcomes over the years.

The assay in step (2) is intended for haplotype tagging SNPs (htSNPs), functional SNPs, and SNPs with Hardy-Weinberg equilibrium P values greater than 0.01 when frequency of minor alleles similar to that of the known Korean database is greater than 5% in the Japanese and Chinese database(there are no recombinants in the Japanese database with linkage disequilibrium(LD) block; WGAViewer, Ge D, Zhang K, Need AC, et al. GenomeRes 2008;18:640-643) as a result of the association analysis on the high-density and high-throughput SNP assay (using the Affymetrix SNP Array 6.0) and the *in vitro* chemosensitivity assay. The reason that the frequency of minor alleles is defined as 5% is to calculate a minimum value of statistical significance that can reduce the number of targeted samples without missing rare mutations.

The linkage disequilibrium (LD) block is caused when a specific genomic region is short to the extent that no crossover has occurred over the generations or when genes are concentrated. Accordingly, the genomic information present in this region is substantially the same and almost preserved and, similarly to this, the haplotype tagging SNPs (htSNPs) refer to a minimum set of SNPs that can identify specific haplotypes from the total haplotypes and represent adjacent SNPs. Accordingly, the LD block SNPs and htSNPs can eliminate the effort of repeatedly assaying SNPs with similar characteristics, thereby reducing the time and expense.

The Hardy-Weinberg equilibrium refers to genetic stability in a random mating population and is usually represented as a P value. When this value is less than 0.01, it represents a deviation from genetic equilibrium caused by inbreeding, genetic drift, mutation, selection, and errors in genetic analysis or selection of population, and thus false positive SNPs resulting from these results can be excluded.

Candidate SNP markers sensitive to 11 anticancer targeted drugs of 11 types of genes discovered in the assay in step 2 are shown in Table 1 of Example 2.

Preferably, the screening method may further comprise the step of performing association analysis on the SNP genotypes selected in step (2) with existing clinical outcomes. The clinical association can be investigated for subjects whose treatment with anticancer targeted drugs has been terminated.

In one instance, the prediction of clinical outcomes of the corresponding patients may be based on the NCCN surveillance guidelines (www.nccn.org), and the determination of chemosensitivity to anticancer drugs may be based on the RECIST criteria (Therasse et al., J Natl Cancer Inst 2000;92:205-16). The observation period for the corresponding patients is an average of 13 months (in a range of 1 to 39 month), which is considered as a sufficient period for the determination of the results considering that these drugs are administered to recurrent and metastatic cancer patients. Through this step, the difference in an *in vivo* drug metabolism environment of the *in vitro* chemosensitivity assay can be excluded, and it is possible to enter directly into a clinical test.

The screening method may further comprise a cell biological assay. If the result of the cell biological assay is the same as the result of the clinical association analysis, the sensitivity of the corresponding SNP to the targeted drug is clearly proven, and even if the results do not coincide exactly, it cannot be said that there is no sensitivity of the corresponding SNP. This is because all sensitive SNPs do not exhibit 100% sensitivity. This includes genetic transformation, cell viability and cytotoxicity assays, and apoptosis assay by caspase-3 Western blot and flow cytometric analysis. As a result, it is possible to develop a biological mechanism for the SNP genotypes of the present disclosure and apply to targeted candidate materials in the development of new drugs.

In one instance, the cell biological assay may comprise the steps of transforming tumor cells with wild-type genes or mutant genes; treating the tumor cells with a particular anticancer drug; and measuring the cell viability of the tumor cells.

The SNPs of the present disclosure will be described in detail below.

A SNP for predicting the sensitivity to bevacizumab, an anticancer targeted drug, comprises an rs2230392 (SNP ID) polynucleotide (SEQ ID NO: 1, Chromosome 17), which is part of ITGA3 (integrin alpha 3) gene, containing a nucleotide at position 201 of the nucleotide sequence and at least 8 contiguous nucleotides, or a complementary polynucleotide thereof. Individuals carrying substitution alleles exhibit greater response to bevacizumab, an anticancer targeted drug, than those with reference alleles.

Bevacizumab is an antibody against VEGF.

A SNP for predicting the sensitivity to bevacizumab and FOLFIRI (5-FU + leucovorin + irinotecan: a complex drug of anticancer drug 5-FU and irinotecan, and leucovorin is used to increase the activity of 5-FU), anticancer targeted drugs, comprises an rs1534443 polynucleotide (SEQ ID NO: 2, Chromosome 6), which is part of UTRN (utrophin) gene, containing a nucleotide at position 406 of the nucleotide sequence and at least 8 contiguous nucleotides, or a complementary polynucleotide thereof. Individuals carrying substitution alleles exhibit greater response to at least one of bevacizumab and FOLFIRI, anticancer targeted drugs, than those with reference alleles.

Another SNP for predicting the sensitivity to bevacizumab and FOLFIRI, anticancer targeted drugs, comprises an rs13321 polynucleotide (SEQ ID NO: 3, Chromosome 9), which is part of TNC (tenascin C) gene, containing a nucleotide at position 251 of the nucleotide sequence and at least 8 contiguous nucleotides, or a complementary polynucleotide thereof. Individuals carrying reference alleles exhibit greater response to at least one of bevacizumab and FOLFIRI, anticancer targeted drugs, than those with substitution alleles.

Still another SNP for predicting the sensitivity to bevacizumab and FOLFIRI, anticancer targeted drugs, comprises an rs1049550 polynucleotide (SEQ ID NO: 4, Chromosome 10), which is part of ANXA11 (annexin All) gene, containing a nucleotide at position 401 of the nucleotide sequence and at least 8 contiguous nucleotides, or a complementary polynucleotide thereof. Individuals carrying substitution alleles exhibit greater response to at least one of bevacizumab and FOLFIRI, anticancer targeted drugs, than those with reference alleles.

A SNP for predicting the sensitivity to bevacizumab and FOLFOX (5-FU + leucovorin + oxaliplatin: a complex drug of anticancer drug 5-FU and oxaliplatin, and leucovorin is used to increase the activity of 5-FU), anticancer targeted drugs, comprises an rs11247226 polynucleotide (SEQ ID NO: 5, Chromosome 15), which is part of LINS1 (lines homolog 1) gene, containing a nucleotide at position 301 of the nucleotide sequence and at least 8 contiguous nucleotides, or a complementary polynucleotide thereof. Individuals carrying substitution alleles exhibit greater response to at least one of bevacizumab and FOLFOX, anticancer targeted drugs, than those with reference alleles.

A SNP for predicting the sensitivity to cetuximab an anticancer targeted drug, comprises an rs2278108 polynucleotide (SEQ ID NO: 6, Chromosome 6), which is part of ZKSCAN3 (zinc finger with KRAB and SCN domains 3) gene, containing a nucleotide at position 401 of the nucleotide sequence and at least 8 contiguous nucleotides, or a complementary polynucleotide thereof. Individuals carrying substitution alleles exhibit greater response to cetuximab, an anticancer targeted drug, than those with reference alleles.

A SNP for predicting the sensitivity to cetuximab and FOLFIRI (5-FU + leucovorin + irinotecan: a complex drug of anticancer drug 5-FU and irinotecan, and leucovorin is used to increase the activity of 5-FU), anticancer targeted drugs, comprises an rs4932305 polynucleotide (SEQ ID NO: 7, Chromosome 15), which is part of SEMA4B (semaphorin 4B) gene, containing a nucleotide at position 201 of the nucleotide sequence and at least 8 contiguous nucleotides, or a complementary polynucleotide thereof. Individuals carrying substitution alleles exhibit greater response to at least one of cetuximab and FOLFIRI, anticancer targeted drugs, than those with reference alleles.

Another SNP for predicting the sensitivity to cetuximab and FOLFIRI, anticancer targeted drugs, comprises an rs2274159 polynucleotide (SEQ ID NO: 8, Chromosome 9), which is part of DFNB31 (deafness, autosomal recessive 31) gene, containing a nucleotide at position 401 of the nucleotide sequence and at least 8 contiguous nucleotides, or a complementary polynucleotide thereof. Individuals carrying substitution alleles exhibit greater response to at least one of cetuximab and FOLFIRI, anticancer targeted drugs, than those with reference alleles.

Still another SNP for predicting the sensitivity to cetuximab and FOLFIRI, anticancer targeted drugs, comprises an rs361863 polynucleotide (SEQ ID NO: 9, Chromosome 22), which is part of ISX (intestine-specific homeobox) gene, containing a nucleotide at position 401 of the nucleotide sequence and at least 8 contiguous nucleotides, or a complementary polynucleotide thereof. Individuals carrying reference alleles exhibit greater response to at least one of cetuximab and FOLFIRI, anticancer targeted drugs, than those with substitution alleles.

A SNP for predicting the sensitivity to cetuximab and FOLFOX (5-FU + leucovorin + oxaliplatin: a complex drug of anticancer drug 5-FU and oxaliplatin, and leucovorin is used to increase the activity of 5-FU), anticancer targeted drugs, comprises an rs3729740 polynucleotide (SEQ ID NO: 10, Chromosome 5), which is part of LIFR (leukemia inhibitory factor receptor alpha) gene, containing a nucleotide at position 201 of the nucleotide sequence and at least 8 contiguous nucleotides, or a complementary polynucleotide thereof. Individuals carrying reference alleles exhibit greater response to at least one of cetuximab and FOLOFX, anticancer targeted drugs, than those with substitution alleles.

Another SNP for predicting the sensitivity to cetuximab and FOLFOX, anticancer targeted drugs, comprises an rs2306393 polynucleotide (SEQ ID NO: 11, Chromosome 12), which is part of MDM1 (Mdm1 nuclear protein homolog), containing a nucleotide at position 301 of the nucleotide sequence and at least 8 contiguous nucleotides, or a complementary polynucleotide thereof. Individuals carrying reference alleles exhibit greater response to at least one of cetuximab and FOLOFX, anticancer targeted drugs, than those with substitution alleles.

The SNP stands for single nucleotide polymorphism, in which the rs is the abbreviation for reference sequence and the following number represents the accession number that identifies each single nucleotide polymorphism provided by a database called dbSNP.

That is, the present disclosure relates to a polynucleotide, or a complement thereof, for predicting the sensitivity to one or more anticancer targeted drugs, selected from polynucleotides comprising at least 8 contiguous nucleotides including each nucleotide at a specific position of the following SEQ ID NOS: 1 to 11:
1. a nucleotide at position 201 of one of SEQ ID NOS: 1, 7, and 10;
2. a nucleotide at position 251 of SEQ ID NO: 3;
3. a nucleotide at position 301 of one of SEQ ID NOS: 5 and 11;
4. a nucleotide at position 401 of one of SEQ ID NOS: 4, 6, 8, 9; and
5. a nucleotide at position 406 of SEQ ID NO: 2.

The at least 8 contiguous nucleotides may preferably be 8 to 100 contiguous nucleotides.

In the present disclosure, the prediction of the sensitivity refers to the prediction of therapeutic effects of a specific anticancer drug administered to a patient. It is expected that high sensitivity to the corresponding anticancer targeted drug provides excellent therapeutic effects, while low sensitivity to the corresponding anticancer targeted drug provides low therapeutic effects. In the present disclosure, the single nucleotide polymorphism (SNP) is a single base-pair variation present in DNA between individuals and is the most abundant type of DNA sequence polymorphism.

The above-mentioned polynucleotides of SEQ ID NOS: 1 to 11 are polymorphic sequences. The polymorphic sequence refers to a nucleotide sequence containing a polymorphic site at which single-nucleotide polymorphism (SNP) occurs. The polynucleotide may be DNA or RNA.

Moreover, the above-mentioned polynucleotides of SEQ ID NOS: 1 to 11 are allele-specific. The allele-specific polynucleotide refers to a polynucleotide specifically hybridized with each allele. That is, the allele-specific polynucleotide has the ability that distinguishes nucleotides of polymorphic sites within the polymorphic sequences of SEQ ID NOS: 1-11 and specifically hybridizes with each of the nucleotides. The hybridization is usually performed under stringent conditions such as at a salt concentration of no more than 1 M and a temperature of 25°C or higher. For example, conditions of 5 x SSPE (750 mM NaCl, 50 mM Na Phosphate, 5 mM EDTA, pH 7.4) and 25 to 30°C may be suitable for allele-specific probe hybridization.

In the present disclosure, the allele-specific polynucleotide may be a primer. As used herein, the primer refers to a single stranded oligonuleotide that acts as a starting point of template-directed DNA synthesis under appropriate conditions in an appropriate solution (e.g., dNTP or dUTP and DNA, RNA polymerase or reverse transcriptase) and at an appropriate temperature. The appropriate length of the primer may vary according to the purpose of use, generally 15 to 30 nucleotides. Generally, a shorter primer molecule requires a lower temperature to form a stable hybrid with a template. Preferably, the 3' end of the primer is aligned with a polymorphic site of SEQ ID NOS: 1 to 11. The primer is hybridized with a target DNA containing a polymorphic site and starts an allelic amplification in which the primer exhibits complete homology with the target DNA. The primer is used in pair with a second primer hybridizing with an opposite strand. Amplified products are obtained by amplification using the two primers, which means that there is a specific allelic form.

In the present disclosure, the allele-specific polynucleotide may be a probe. As used herein, the probe refers to a hybridization probe, that is, an oligonucleotide capable of sequence-specifically binding with a complementary strand of a nucleic acid. The probe according to the present disclosure is an allele- specific probe. In this regard, when there are polymorphic sites in nucleic acid fragments derived from two members of the same species, the probe is hybridized with DNA fragments derived from one member but is not hybridized with DNA fragments derived from the other member. In this case, hybridization conditions should be stringent enough to allow hybridization with only one allele by significant difference in hybridization strength between alleles. Preferably, the central portion of the probe (e.g., position 7 for a 15 nucleotide probe, or position 8 or 9 for a 16 nucleotide probe) is aligned with each polymorphic site of the nucleotide sequences of SEQ ID NOS: 1 to 11. As a result, a significant difference in hybridization between alleles may be caused.

Another aspect of the present disclosure relates to a polynucleotide comprising the SNP for predicting the sensitivity to a specific anticancer targeted drug as described herein and a microarray comprising a polypeptide encoded by the same or a cDNA thereof. The microarray according to the present disclosure may be prepared using the SNP for predicting the sensitivity to an anticancer drug by a common method known in the art.

For example, the polynucleotide may be immobilized on a substrate coated with an active group selected from the group consisting of amino-silane, poly-L-lysine, and aldehyde. Moreover, the substrate may be selected from the group consisting of a silicon wafer, glass, quartz, metal, and plastic. The immobilization of the polynucleotide on the substrate may be performed by micropipetting using a piezoelectric method or by using a spotter in the shape of a pin.

Still another aspect of the present disclosure relates to a kit for predicting the sensitivity to an anticancer targeted drug, comprising the microarray as described herein.

The kit according to the present disclosure may further comprise a primer set used to isolate and amplify DNA including the corresponding SNP from a subject in addition to the microarray as described herein. A suitable primer set may be easily designed by those skilled in the art with reference to the sequences described herein.

Yet another aspect of the present disclosure relates to a method for predicting the sensitivity to an anticancer drug using the SNP for predicting the sensitivity to an anticancer drug as described herein.

The method comprises the steps of:
(1) isolating nucleic acid molecules from a subject; and
(2) determining the type of a nucleotide at the position of SNP of a polynucleotide comprising SEQ ID NOS: 1 to 11 from the isolated nucleic acid molecules.

For example, DNA is isolated from the patient's tissues, fluids, or cells, amplified by PCT, and subjected to SNP analysis. The SNP analysis may be performed by a common method known in the art. For example, the SNP analysis may be performed using a real time PCR system or by direct determination of the nucleotide sequence of the nucleic acid by a dideoxy method. Otherwise, the SNP analysis may be performed by hybridizing the DNA with a probe containing the sequence of the SNP site or a complementary probe thereof and examining the degree of the hybridization. Moreover, the SNP analysis may be performed by a method selected from the group consisting of allele-specific probe hybridization, allele-specific amplification, sequencing, 5' nuclease digestion, molecular beacon assay, oligonucleotide ligation assay, size analysis, and single-stranded conformation polymorphism.

In the prediction method, when the nucleotide at the position of the SNP, corresponding to the nucleotide at position 201 of SEQ ID NO: 1, is determined as A (A/A or G/A heterozygous genotype) in step (2), it may be predicted that the sensitivity to bevacizumab, an anticancer targeted drug, is high.

When the nucleotide at the position of the SNP, corresponding to the nucleotide at position 406 of SEQ ID NO: 2, is determined as T (T/T or C/T heterozygous genotype), it may be predicted that the sensitivity to at least one of bevacizumab and FOLFIRI (5-FU + leucovorin + irinotecan), anticancer targeted drugs, is high.

When the nucleotide at the position of the SNP, corresponding to the nucleotide at position 251 of SEQ ID NO: 3, is determined as C (C/C), it may be predicted that the sensitivity to at least one of bevacizumab and FOLFIRI (5-FU + leucovorin + irinotecan), anticancer targeted drugs, is high.

When the nucleotide at the position of the SNP, corresponding to the nucleotide at position 401 of SEQ ID NO: 4, is determined as T (T/T), it may be predicted that the sensitivity to at least one of bevacizumab and FOLFIRI (5-FU + leucovorin + irinotecan), anticancer targeted drugs, is high.

When the nucleotide at the position of the SNP, corresponding to the nucleotide at position 301 of SEQ ID NO: 5, is determined as G (G/G or A/G heterozygous genotype), it may be predicted that the sensitivity to at least one of bevacizumab and FOLFOX (5-FU + leucovorin + oxaliplatin), anticancer targeted drugs, is high.

When the nucleotide at the position of the SNP, corresponding to the nucleotide at position 401 of SEQ ID NO: 6, is determined as G (G/G or C/G heterozygous genotype), it may be predicted that the sensitivity to cetuximab, an anticancer targeted drug, is high.

When the nucleotide at the position of the SNP, corresponding to the nucleotide at position 201 of SEQ ID NO: 7, is determined as A (A/A or G/A heterozygous genotype), it may be predicted that the sensitivity to at least one of cetuximab and FOLFIRI (5-FU + leucovorin + irinotecan), anticancer targeted drugs, is high.

When the nucleotide at the position of the SNP, corresponding to the nucleotide at position 401 of SEQ ID NO: 8, is determined as G (G/G), it may be predicted that the sensitivity to at least one of cetuximab and FOLFIRI (5-FU + leucovorin + irinotecan), anticancer targeted drugs, is high.

When the nucleotide at the position of the SNP, corresponding to the nucleotide at position 401 of SEQ ID NO: 9, is determined as C (C/C), it may be predicted that the sensitivity to at least one of cetuximab and FOLFIRI (5-FU + leucovorin + irinotecan), anticancer targeted drugs, is high.

When the nucleotide at the position of the SNP, corresponding to the nucleotide at position 201 of SEQ ID NO: 10, is determined as G (G/G), it may be predicted that the sensitivity to at least one of cetuximab and FOLFOX (5-FU + leucovorin + oxaliplatin), anticancer targeted drugs, is high.

When the nucleotide at the position of the SNP, corresponding to the nucleotide at position 301 of SEQ ID NO: 11, is determined as T (T/T or T/C heterozygous genotype), it may be predicted that the sensitivity to at least one of cetuximab and FOLFOX (5-FU + leucovorin + oxaliplatin), anticancer targeted drugs, is high.

In one instance, as a result of performing clinical association analysis on subjects treated with bevacizumab or cetuximab, the subjects carrying reference alleles such as DFNB31 rs2274159, LIFR rs3729740, and ISX rs361863 and substitution alleles such as ANXA11 rs1049550, ITGA3 rs2230392, and LINS1 rs11247226 exhibited greater response to anticancer drugs or longer progression-free survival (Example 3).

The following Examples are given for illustrating the present invention in more detail, and it will be understood by those skilled in the art to which the present invention pertains that the scope of the present invention is not limited by the following examples.

### Example 1: Step (1) to determine SNP genotypes

Basic data of SNP genotypes sensitive to anticancer targeted drugs were obtained by selecting 56 SNPs having a linkage disequilibrium (R²) value of greater than 0.8 from a total 4,163 SNPs having a -Log (P) value of greater than 3 by performing association analysis on the result of a high-density and high-throughput SNP assay (using the Affymetrix SNP Array 6.0) and the result of an *in vitro* chemosensitivity assay in 118 colorectal cancer patients.

### Example 2: Step (2) to determine SNP genotypes

Candidate SNP genotypes exhibiting a frequency of 10% or higher (www.hanpmap.org) in existing Japanese and Chinese analysis data and exhibiting linkage disequilibrium were selected from the SNP genotypes selected in step (1), and further 15 candidate SNPs were discovered by non-synonymous, haplotype-tagging, and functional SNP selection (FIG. 1).

Then, 11 SNPs without significant deviation from Hardy-Weinberg equilibrium (P>0.01) in the same SNP genotype assay using lymphocyte DNA samples in 480 normal populations were selected as candidates for clinical association analysis for the final assay in an Example of step (3).

**Table 1: Candidate SNPs of 11 genes discovered in step (2) sensitive to 11 anticancer drugs**

| Regimens | Genes | SNP ID | Alleles | | Results |
|---|---|---|---|---|---|
| | | | Reference | Substitution | |
| Bevacizumab | ITGA3 | rs2230392 | G | A | A>T |
| Bevacizumab + FOLFIRI* | UTRN | rs1534443 | C | T | S>N |
| Bevacizumab + FOLFIRI* | TNC | rs13321 | C | G | E>Q |
| Bevacizumab + FOLFIRI* | ANXA11 | rs1049550 | C | T | R>C |
| Bevacizumab + FOLFOX* | LINS1 | rs11247226 | A | G | I>V |
| Cetuximab | ZKSCAN3 | rs733743 | C | G | R>T |
| Cetuximab + FOLFIRI* | SEMA4B | rs4932305 | G | A | I>M |
| Cetuximab + FOLFIRI* | DFNB31 | rs2274159 | G | A | A>V |
| Cetuximab + FOLFIRI* | ISX | rs361863 | C | T | S>G |
| Cetuximab + FOLFOX* | LIFR | rs3729740 | G | A | D>N |
| Cetuximab + FOLFOX* | MDM1 | rs2306393 | T | C | H>R |

| | | | | | |
|---|---|---|---|---|---|
| * FOLFOX, FL (5-FU/leucovorin) + irinotecan; FOLFOX, FL (5-FU/leucovorin) + oxaliplatin | | | | | |

### Example 3: Association analysis step between SNP genotypes and existing clinical outcomes

Clinical association analysis was performed on 69 subjects treated with bevacizumab and 67 subjects treated with cetuximab, whose progress of treatment with the corresponding anticancer drugs were confirmed, to investigate the clinical association. The prediction of clinical outcomes of the corresponding patients was based on the NCCN surveillance guidelines (www.nccn.org), and the determination of chemosensitivity to anticancer drugs were based on the RECIST criteria (Therasse et al., J Natl Cancer Inst 2000;92:205-16). The observation period for the corresponding patients was an average of 13 months (in a range of 1 to 39 month), which was considered as a sufficient period for the determination of the results when considering that these drugs were administered to recurrent and metastatic cancer patients.

### 3-1: Clinical association analysis related to survival on subjects treated with anticancer targeted drugs

Association analysis was performed on the SNP genotypes sensitive to the individual anticancer drugs obtained in steps (1) and (2) in colorectal cancer patients whose treatment with the corresponding drugs was terminated. First, the association analysis was performed on the candidate SNP genotypes sensitive to the respective drugs in 69 patients treated with bevacizumab and 67 patients treated with cetuximab, including 98 recurrent and metastatic cancer patients with crossover treatment. Patients treated with bevacizumab, an anticancer targeted drug, and carrying homozygous or heterozygous substitution alleles of ITGA3 rs2230392 SNP genotype exhibited significantly longer mean progression-free survival and overall survival than those with homozygous reference alleles (PFS, 8.7 ± 0.7 months vs. 5.7 ± 0.7 months, P = 0.018; OS, 22.5 ± 2.1 months vs. 13.4 ± 1.9 months, P = 0.006) (FIG. 2). On the contrary, patients treated with cetuximab, an anticancer targeted drug, and carrying homozygous reference alleles of ISX rs361863 SNP genotype exhibited significantly longer mean progression-free survival than those with homozygous or heterozygous substitution alleles (PFS, 8.5 ± 1.2 months vs. 4.4 ± 0.5 months, P = 0.046) (FIG. 3).

### 3-2: Clinical association analysis of drug response on subjects treated with anticancer targeted drugs(Table 2)

As a result of performing clinical association analysis with respect to drug response on the SNP genotypes selected in steps (1) and (2), respectively, in 69 subjects treated with bevacizumab and 67 subjects treated with cetuximab, the subjects treated with bevacizumab and carrying homozygous substitution alleles of ANXA11 rs1049550 and homozygous or heterozygous substitution alleles of LINS1 rs11247226 exhibited a significantly high disease-control response rate(stable disease + partial disease + complete response) (P = 0.025, 0.019). Meanwhile, the subjects treated with cetuximab and carrying homozygous reference alleles of DFNB31 rs2274159 and LIFR rs3729740 exhibited significantly high objective response rate(partial disease + complete response) and disease-control response rate(P = 0.023, 0.049).

**Table 2: Sensitivity to anticancer targeted drugs in metastatic cancer patients based on candidate SNP genotypes**

| Genes | SNP IDs | Genotypes | Regimens | No. with disease-control response (DCR) or objective response (OR)/ total patients (%) | | |
|---|---|---|---|---|---|---|
| | | | | Types | Responses | P-values |
| ANXA11 | rs1049550 | CC+CT | bevacizumab | DCR | 16/32 (50) | 0.025 |
| | | TT | | | 28/37 (76) | |
| LINS1 | rs11247226 | AA | bevacizumab | DCR | 25/46 (54) | 0.019 |
| | | AG+GG | | | 19/23 (83) | |
| DFNB31 | rs2274159 | GG | cetuximab | OR | 8/17 (47) | 0.023 |
| | | GA+AA | | | 9/50 (18) | |
| LIFR | RS3729740 | GG | cetuximab | DCR | 30/42 (71) | 0.049 |
| | | GA+AA | | | 12/25 (48) | |

## Claims

1. A method for predicting sensitivity of a subject having colorectal cancer to anticancer targeted drugs selected from at least one of bevacizumab and FOLFIRI (5-FU + leucovorin + irinotecan), the method comprising:
determining the type of a nucleotide at the position of the SNP corresponding to the nucleotide at position 401 of SEQ ID NO: 4 (which is part of the ANXA11 (annexin All) gene) from nucleic acid molecules isolated from the subject.

2. The method of claim 1, wherein, when the nucleotide at the position of the SNP, corresponding to the nucleotide at position 401 of SEQ ID NO: 4, is determined as T, it is predicted that the sensitivity to at least one of bevacizumab and FOLFIRI (5-FU + leucovorin + irinotecan), anticancer targeted drugs, is high.

3. Use of a polynucleotide or a complementary polynucleotide thereof, selected from polynucleotides comprising at least 8 contiguous nucleotides including a nucleotide at position 401 of SEQ ID NO: 4, in a method according to claim 1 or 2.

4. Use of a polynucleotide or a complementary polynucleotide thereof according to claim 3 wherein the at least 8 contiguous nucleotides are 8 to 100 contiguous nucleotides.

5. Use of a primer or probe in a method according to claim 1 or 2 wherein the primer or probe comprises the polynucleotide or complementary polynucleotide thereof defined in claim 3 or 4.

6. Use of a microarray, or a kit comprising said microarray, in a method according to claim 1 or 2, wherein the microarray comprises the polynucleotide defined in claim 3 or 4, a polypeptide encoded by the same, or a cDNA thereof.

## Patentansprüche

1. Verfahren zum Vorhersagen der Empfindlichkeit eines Subjekts mit Dickdarmkrebs gegenüber Antikrebsmedikamenten, die ausgewählt sind aus mindestens einem aus Bevacizumab und FOLFIRI (5-FU + Leucovorin + Irinotecan), wobei das Verfahren Folgendes umfasst:
Bestimmen des Typs eines Nukleotids an der Position des SNP, das dem Nukleotid an Position 401 von SEQ ID NO: 4 (die Teil des ANXA11- (Annexin A11) Gens ist) entspricht, aus Nukleinsäuremolekülen, die aus dem Subjekt isoliert wurden.

2. Verfahren nach Anspruch 1, wobei, wenn das Nukleotid an der Position des SNP, das dem Nukleotid an Position 401 aus SEQ ID NO: 4 entspricht, als T bestimmt wird, vorhergesagt wird, dass die Empfindlichkeit gegenüber mindestens einem der Krebsmedikamente aus Bevacizumab und FOLFIRI (5-FU + Leucovorin + Irinotecan) hoch ist.

3. Verwendung eines Polynukleotids oder eines komplementären Polynukleotids davon, ausgewählt aus Polynukleotiden, umfassend mindestens 8 aufeinanderfolgende Nukleotide einschließlich eines Nukleotids an Position 401 aus SEQ ID NO: 4, in einem Verfahren nach Anspruch 1 oder 2.

4. Verwendung eines Polynukleotids oder eines komplementären Polynukleotids davon nach Anspruch 3, wobei die mindestens 8 aufeinanderfolgenden Nukleotide 8 bis 100 aufeinanderfolgende Nukleotide sind.

5. Verwendung eines Primers oder einer Sonde in einem Verfahren nach Anspruch 1 oder 2, wobei der Primer oder die Sonde das in Anspruch 3 oder 4 definierte Polynukleotid oder komplementäre Polynukleotide davon umfasst.

6. Verwendung eines Microarrays oder eines das Microarray umfassenden Kits in einem Verfahren nach Anspruch 1 oder 2, wobei das Microarray das in Anspruch 3 oder 4 definierte Polynukleotid, ein von Selbigem kodiertes Polypeptid oder eine cDNA davon umfasst.

## Revendications

1. Procédé permettant de prédire la sensibilité d'un sujet atteint d'un cancer colorectal à des médicaments anticancéreux ciblés choisis parmi au moins un médicament entre le bévacizumab et le FOLFIRI (5-FU + leucovirine + irinotécane), le procédé comprenant l'étape consistant à :
déterminer le type d'un nucléotide à la position du SNP correspondant au nucléotide à la position 401 de la SEQ ID n° : 4 (qui fait partie du gène ANXA11 (annexine A11)) à partir de molécules d'acides nucléiques isolées provenant du sujet.

2. Procédé selon la revendication 1, dans lequel, lorsque le nucléotide à la position du SNP, correspondant au nucléotide à la position 401 de la SEQ ID n° : 4, est déterminé comme étant T, il est prédit que la sensibilité à au moins un parmi le bévacizumab et le FOLFIRI (5-FU + leucovirine + irinotécane), des médicaments anticancéreux ciblés, est élevée.

3. Utilisation d'un polynucléotide ou d'un polynucléotide complémentaire de celui-ci, choisi parmi les polynucléotides comprenant au moins 8 nucléotides contigus comportant un nucléotide à la position 401 de la SEQ ID n° : 4, dans un procédé selon la revendication 1 ou 2.

4. Utilisation d'un polynucléotide ou d'un polynucléotide complémentaire de celui-ci selon la revendication 3, lesdits au moins 8 nucléotides contigus étant 8 à 100 nucléotides contigus.

5. Utilisation d'une amorce ou d'une sonde dans un procédé selon la revendication 1 ou 2, ladite amorce ou ladite sonde comprenant le polynucléotide ou un polynucléotide complémentaire de celui-ci défini dans la revendication 3 ou 4.

6. Utilisation d'une puce à ADN, ou d'un kit comprenant ladite puce à ADN, dans un procédé selon la revendication 1 ou 2, ladite puce à ADN comprenant le polynucléotide défini dans la revendication 3 ou 4, un polypeptide codé par celui-ci ou un ADNc de celui-ci.


<110> THE ASAN FOUNDATION Korea Research Institute of Bioscience and Biotechnology
<120> SNP for predicting sensitivity to an anti-cancer targeted agent
<150> KR10-2010-0056279
   <151> 2010-06-15
<160> 11
<170> KopatentIn 1.71
<210> 1
   <211> 401
   <212> DNA
   <213> Homo sapiens
<400> 1
   gatggagaga aagggggaaa gggaggcaga ggtggggtgg ggaggtagag acccctcacc      60
   cagaatagga ggaggaggag aaggccagaa agccaactag aatagtgtgc atcccctcct     120
   tccccatgac atcacccccg ccaggcccca tatgccactc tctgcctccc tgacccttgt    180
   ggcagatgga gctgctcatc rcctttgagg tcatcggggt gaccctgcac acaagggacc     240
   ttcaggtgca gctgcagctc tccacgtgag tgacctcgaa aagccagtct gggtcagggc     300
   tgaggtatct tggatcacct tcctaacccc tgcatttcct cccaaggtcg agtcaccagg     360
   acaacctgtg gcccatgatc ctcactctgc tggtggacta t                         401
<210> 2
   <211> 1351
   <212> DNA
   <213> Homo sapiens
<400> 2
   aaacctgatc atatcacaac agtataaatg caagaaaagc tctatgcttt gtagttagcc      60
   ctgaatggtt gtttgacaga tacagaattt tttaaacctc attctctcat agtttaggat     120
   aaacaaaagg gattcaaaca gatctggtct acctctgggt tgtggcgttt ccatgacaag     180
   cttttacact atgcacctac tttcattcgg gaaacggtct tattgatctc ttctacatcc     240
   ccaacagtga caatgttgga cttcagcatc tggtcgatta ataccagcca gtcggctagt     300
   tctgttatag ttttatcaag atcagcagga attgaaattt ccgaagtacg atgagactga     360
   acaggaatat ctgacgcaga tgatactagc accacctttt ggacaytagg atgagctgaa     420
   atgaaaaatg tagcaaaaga gaaattaatc aacaggatgt tttaattatg gtggtgtcat     480
   ggtgacacgt gtatgtgtat ttaaataaaa tttagtttaa gtaaaaaaaa ttctaagagg     540
   ttattttttc atactgaaat ggctatggta ttacctgtaa tatccatatg tctttggtag     600
   ctatcaagcc aacaaaacag ttttgcaaca ttctataaca taatcagcac tgacactgag     660
   aaaaattaaa agtacaaatg tgggagaact ggaagtatta ttttattttg gcaagatgat     720
   tttaattata aagccaactt attcataatt ttttattttt ttcatttaac tttataatgt     780
   gagcatattt tattcttagt aaacacaatt atgaatacct atgtaataac ctatacccat 840
   attcactttt atttaaccct tggatattta gattgctcct gaaattttat tttaattttt 900
   taaaaaactt gcaaagaata tcttttcatg tagagtattt tgcacatttc tgatctctat 960
   cttagcatag atttcccaag tagaattact gagtcagaaa atacaaaaaa aagtatgtat 1020
   ttaatattca ttgccaaatt gattggtttt gatacaataa gtagttgaat tagtaattaa 1080
   aactatctgt aacctacttc aggtatgata tattagatga ggcttaaata attttctaaa 1140
   atgtatagcc aatctgtaaa ttcagcacaa tcctaataaa aatcccaaca ggcttgaatg 1200
   taacaaaatc attaattcaa atttctggtt ataaaattaa tattgaagag aaaaggacaa 1260
   aaaacagtca aattattttt tgatagagga atatgccctc ccagatatta agttatatta 1320
   tttagctaat aattattaac tcagtatatt a 1351
<210> 3
   <211> 501
   <212> DNA
   <213> Homo sapiens
<400> 3
   gcccatttct atgagttttt aattgtttgc atcacattat tgcacctgga aattccccag 60
   gcattctagt ccatagagtt aagctaagaa ttaagctcca gctcagtcct cctgcactga 120
   gagcatcacc atttctcctt attccacagt tggactcctg taccccttcc ccaaggactg 180
   ctcccaagca atgctgaatg gagacacgac ctctggcctc tacaccattt atctgaatgg 240
   tgataaggct saggcgctgg aagtcttctg tgacatgacc tctgatgggg gtggatggat 300
   tgtgagtacc accagggact gcagagccct caggcctagg ggaagaggga gagggcaaag 360
   caggggagga gaagagggtg atctcctcct gatgcactct gacaccccca tgtgtgaggc 420
   cttgcaagat agtgcctgtc acacagaggc actcaggaat tctctcttaa acaaaagaat 480
   gggaaacacg ctggctactt t 501
<210> 4
   <211> 801
   <212> DNA
   <213> Homo sapiens
<400> 4
   cgacatctta cctgagactg tttgtccgag tagtttggaa gccgaggcac catcactgat        60
   gctcccggct ttgaccccct gcgagatgcc gaggtcctgc ggaaggccat gaaaggcttc      120
   ggtaagagac cctgggtggc tcaaatccta ctccctgccc cctatttccc aggcagtttc      180
   actgtggctc tgtgggctgg ggtagctggg ggacgctgac ctggccccag tggtgataga      240
   aggtggatgt gggggtatgg ctagcatgcc cagaacagtt gaggggccca ggccagggaa      300
   gtctcagctg cagagcatct catgacccct acccgaccca cccctgctgc ctcttcctcc      360
   agggacggat gagcaggcca tcattgactg cctggggagt ygctccaaca agcagcggca      420
   gcagatccta ctttccttca agacggctta cggcaaggcg agctgcgggg tgggggcgcg      480
   ggacagtgag gcgtgtcctg ggctcagcac agccctgctc tcccctgctt ttccttgggt      540
   gggcccggat ctcccggatg gacagtaagg agcaggaggg catttccttc tgcccatcca      600
   gctggtggca tcgttgggag gtgaaggatg ccaccgggtg ctgaggtttg ctgagaggct      660
   ccagacatct atccctgggt tctagccctt gctctgccac atctcactga ggttgtggcc      720
   acatcatttt ccttcccctg ccctcttcat gaccaccctg gggggtttag ctgatgtaag      780
   agctttgcag ggaaaggctg t                                                801
<210> 5
   <211> 601
   <212> DNA
   <213> Homo sapiens
<400> 5
   gcagtcagca tcccctttaa cttgagagac ttagtgatta agtggaaaca gttgctttct        60
   cttgtaataa gcatatagca gttagaattt ttaaattatt tttcctattt taggtttcac      120
   tgaagaaact tcttagagat tcattgcact tctgagattt aatgtttaca acttggagtt      180
   gtcgaccttc ttataagata cattttggaa gtcaaaatga aagttttctg tgaagtttta      240
   gaagagttat acaagaaggt acttcttgga gccacacttg aaaatgacag ccatgattac      300
   rtcttttatc tcaacccagc agtttcagat caagattgtt ctacagccac ctccttagaa      360
   tgggcaaaca cctgtggtat ccagggcagg catcagccca tctctgttgg tgtggctccc      420
   attgctgtag cacctgtgtg tttgaagacc aactctcaga tgagcggttc cagagaagta      480
   atgctccttc agttaacagt gatcaaagtg atgacaaccc ggatattgtc tgtcaaaacc      540
   gagttccatg caaaggagca gtacagagat gtaattaaaa ttctcttaga atcagccaaa      600
   g                                                                      601
<210> 6
   <211> 801
   <212> DNA
   <213> Homo sapiens
<400> 6
   ttctacctgc ggcgccggct catccagctg cctctccaaa tactccaata gcaccaccac      60
   ctcctccccg ctctctggat gctgctcccg cacccagctc tgcagattcc ccggcaggat     120
   ggtcaggaac tgctccagca ccagcagctc caggatctgc tccttgctgt gcatctcagg     180
   ctgcagccac tgtcggcaga gctctcggag ccgactcagc gcctcgcggg ggcctgcagc     240
   ctccgggtag cggaagcctc ggaagcgctc gcgggagccc tcagaaccca gatctgggct     300
   actgggaaaa ccggcttctt cttcctccac ctttatgacc agaagctcca tctggtcttc     360
   tgtagactgg gcatccaggg ctgtgctttc acttaattct stagccatcc taggccaagg     420
   cagtactcag gcctcactct agcttccagc gctatttctg cagaagatcc ctgaaaggtg     480
   ggaaatcatt attaaccagt aaacttgcaa gtagtccctt ctctcctgcc caggagccca     540
   gagacatctc tggggaactg ctgcagaaat ttctccatgt tcagtggttc tgcagcttaa     600
   tcctctacac caccccaaac ctgaatccca tagtaaggtt cccaaaggat gtcctggagt     660
   gatcctggac acccccaagc ccctgactaa tcaggaaaaa aactgaaatt ggtgaggatc     720
   cagggcactt cctggactct ctaggaagct cctctcagtg acttgctcag agttatccca     780
   ttaccaacca ggtctgtggt t                                               801
<210> 7
   <211> 401
   <212> DNA
   <213> Homo sapiens
<400> 7
   cgactgtcca ggttcaaacc cttcggcacc gtgtcctggc tgaggaggac ggcgtccctg      60
   acacctgacc ccttctgtcc cacacacctc acctgcctgc agagttgcct tctcctgcac     120
   gcctctctct ttctcaccaa gcctgctcct cattccctgg agtgcccctg agcccatgtg     180
   tccaccctcc tgcagaacat rgagaacttc accctggcaa gggacgagaa ggggaatgtc     240
   ctcctggaag atggcaaggg ccgttgtccc ttcgacccga atttcaagtc cactgccctg     300
   gtggttggtg agtgttgagg gcaggatggc ttcattgagg ttccatgtag gggcacacag     360
   ggctagaggg ctagctttgg acaggctgag tccatgccat g                         401
<210> 8
   <211> 801
   <212> DNA
   <213> Homo sapiens
<400> 8
   tctggtggtg gcatcaggac ttgaatcgaa gcctgactcc gaagactgtc tcagccacct      60
   ggccatgtca aaactgagag gagcttaaag attcccaaga ccaatctcag cctcagcccc     120
   tagtgtcagt gcaaaggcag agctgaaact gggcctccag attcctaatc cagtgctcct     180
   ccaggacgtc caaatccctc cagttatagg gagctcacta cctccctttg tggtcacttg     240
   ccaagctgag ccctggtccc gggacctccc attctgtgcc tggggcccgt gttcctctca     300
   gccctctgca tcctcctcct cctggctcac cggtttgttg gccccatctg tgggcctctc     360
   gttccgaggc agctccttgc tactcctgct cttggtggac rccgactgcc ttcctcggcc     420
   tggggcgctg gcctctgcct cgccagcatc cacaccactg tcctcgctta gagtctgccc     480
   gctgtccgag aggtgggaga gcgtagaggc tgctggagag gggaaaagga agagagaagc     540
   agcttatata gctggggtca gggagggctg cctggaggca gtgacggaga tggaggctgg     600
   aaggctgggg gaattcccca ggcaaagatg ggggaaggaa tgaagagatc gtggggccag     660
   agggcaggtc cactccctgc caccctctcc acctactcaa gacctgtcct ttcaaccaga     720
   cctggtcttg aaggctgtga cttccaggac atctgcctgg atctcccagt tcccagccac     780
   tggcataatg ataaaacaga g                                                801
<210> 9
   <211> 801
   <212> DNA
   <213> Homo sapiens
<400> 9
   cggggcctgg ttacaataac aagccccagg tccctgcatt ctgcaactaa ctgagccttg        60
   gtgtctgaag ctgaaaagca cagttttggg ttagattgaa aggtatatca ggaaaccagc       120
   ccagagccag gtcttgctct gaattttaga gtcctctatc cagttaatat ggacggagaa       180
   gctttttctc tgcctgagcc cagctgggtc ttggagacca ggaaacagaa agaaatgatc       240
   agaagacact taccctgggg ctggtccttt ggaggctttt ctagcccaga gcctgaggcc       300
   gcagcttctc cggggccctc ttcacctggc ccttctggtc tgtccatatc actcctcctg       360
   gcaggcctct ttaggatcgc ctcaatggag aaggacaggc ycagcttctt cggggcctca       420
   cagcacccca agctatttct ctccataccc ctgcagagag cagggcccac ctcagcacac       480
   attgaggggc tcaggggtga ctctgtgtag gccaaggagg gtgccagtgg agagtggcgt       540
   acttcctccc ctgaacctcc tggggtcaag agggcgcgtg gaggtgaaga acagagagcc       600
   tccagttaga tgatccaaac aggatttcgt gtccacaggg acttccatgg gcaggtcaca       660
   gcagaagagc tggaaggagt ggtatcctgt gcccacggct cagcccttac cagtttgaca       720
   tcaaaggagt caccatggct gaattggggt tcaatcttcc agagaagggt cttctcggac       780
   acagttgcct gcacaatcac c                                                 801
<210> 10
   <211> 401
   <212> DNA
   <213> Homo sapiens
<400> 10
   gtgctggaat tataagcgtg agtcaccacg cctggccaac cttgttattg ttaatatagt        60
   gtccagttta aacccaatta ccttgcctag tcacagtcac gttttgcagg gttttataac       120
   aatttgtctg tttttcatag cctttaccca ttaatgaagc taatggaaaa atactttcct       180
   acaatgtatc gtgttcatca ratgaggaaa cacagtccct ttctgaaatc cctgatcctc       240
   agcacaaagc agagatacga cttgataaga atgactacat catcagcgta gtggctaaaa       300
   attctgtggg ctcatcacca ccttccaaaa tagcgagtat ggaaattcca aatggtgagt       360
   gcttgagatg atttagtata taagaacggg aaactaaagt g                           401
<210> 11
   <211> 601
   <212> DNA
   <213> Homo sapiens
<400> 11
   attataggac gtaaatgaaa ataatcctaa ctacttcctc ccaaagtagg ccagaattca        60
   agtattcaat taccaatcac tagtgactta aatacctgag tgaatcaaat tattcaccat       120
   acagcagtgc ctcctttgtg acattgaaat aaaatctgca atttattagt cataaaatca       180
   aaagagtaaa ccactatatt tagtgacaaa atacatatca accatgtaaa taacgcttct       240
   ctatactgag taatttaaaa ctctcaccct tctttcatct tatctgcctt agatttctca       300
   ygtacatcca acttctcttg ctctcccata aaagcctgct tgcccgtttt cctgtcccct       360
   tcctcttcat tgtcgtcctc ctcctctttc tccccgggct gtttctgtac gtcttcactt       420
   gtgttctctg tatcccaagc cagccgcctt ctaacgggta tggtgggagc tgacacaccc       480
   aatttctccg tggtattttt ctggggctgt tcttccacga tatttccttt ttcttctggt       540
   tctgtagaag gacatttctg caaagtctta tggcttgtag gatctctgcg taacaaaatc       600
   a                                                                       601